**Europäisches Patentamt**

**European Patent Office**

**Office européen des brevets**

(19)

(11) Numéro de publication : **0 506 559 A1**

# (12) DEMANDE DE BREVET EUROPEEN

(21) Numéro de dépôt : **92400819.6**

(22) Date de dépôt : **25.03.92**

(51) Int. Cl.⁵ : **A61B 6/14,** G02B 6/42

(30) Priorité : **25.03.91 FR 9103591**

(43) Date de publication de la demande :
**30.09.92 Bulletin 92/40**

(84) Etats contractants désignés :
**DE FR IT SE**

(71) Demandeur : **TROPHY-RADIOLOGIE**
**106 Rue de la Jarry**
**F-94300 Vincennes (FR)**

(72) Inventeur : **Stoichita, Catalin**
**37 Avenue Louis Blanc**
**F-94210 La Varenne (FR)**

(74) Mandataire : **Thinat, Michel et al**
**Cabinet Weinstein, 20 Avenue de Friedland**
**F-75008 Paris (FR)**

(54) **Système permettant d'obtenir des images radiologiques notamment dentaires.**

(57)    L'invention concerne un système permettant d'obtenir des images radiologiques.

Le système est caractérisé en ce qu'il comprend un ordinateur pour applications graphiques (1), une source de rayons X (2) à paramètres d'émissions contrôlables par l'ordinateur, un diaphragme de rayons X (3), variable et adapté pour être commandé par l'ordinateur pour limiter le faisceau de rayons X au champ nécessaire, une mire (4) pour rayons X, déplaçable et gérée par l'ordinateur (1) pour établir les paramètres du diaphragme (3) et un capteur (7) de rayons X, adapté pour recevoir les images de rayons X de l'objet à analyser (5), pour convertir les informations d'images en informations numériques à l'ordinateur (1).

L'invention trouve application dans le domaine médical dentaire.

EP 0 506 559 A1

L'invention concerne un système permettant d'obtenir des images radiologiques notamment dentaires.

Elle constitue en fait un perfectionnement à l'appareil décrit dans la demande de brevet français N°2 578 737.

L'appareil décrit dans la demande de brevet ci-dessus comprend un générateur de rayons X extra-buccal destiné à irradier une dent, un capteur intra-buccal des rayons X ayant traversé la dent derrière laquelle il est positionné et une unité de traitement électronique extra-buccale enregistrant les informations électriques en sortie du capteur intra-buccal auquel elle est reliée afin de faire apparaître l'image de la dent sur le moniteur d'une chaîne de visualisation. Le capteur intra-buccal est constitué de la puce d'un dispositif à transfert de charge ou éléments CCD de caméra collée sur la face du plan tronqué d'un écran adoptant la configuration générale d'une pyramide tronquée dont le plan de base est recouvert d'un scintillateur qui transforme les rayons X émergeant de la dent irradiée en rayons du spectre visible qui sont enregistrés par la puce. L'écran est formé de fibres optiques en verre doppé qui jouent un double rôle : celui d'écran contre les rayons X pour protéger la puce de caméra CCD située en arrière et celui d'objectif photo à petites dimensions, c'est-à-dire pour réduire l'image visible obtenue sur le film scintillateur à l'entrée.

Le type de capteur intra-buccal des rayons X ci-dessus connu peut présenter plusieurs inconvénients parmi lesquels :

1) aberrations géométriques possibles dûes aux fibres optiques réductrices en particulier sur les bords de l'image;

2) existence possible d'un moirage, dûe à la double discrimination de l'image par la puce de caméra CCD et par le réseau de fibres optiques réductrices (dénommé dans la demande ci-dessus référencée par "effet de double moirage");

3) non-uniformité possible de sensibilité sur la surface du capteur dûe à la disposition insuffisamment régulière des fibres,

4) mauvaise influence possible sur les paramètres de bruit et de sensibilité que le collage des fibres sur la puce de caméra CCD peut apporter à cause des contraintes mécaniques occasionnées par le durcissement de la colle et/ou de la forte activité chimique à l'interface colle-CCD ou d'autres détails de la technologie du collage;

5) existence d'un cablage complexe pour le transport des signaux de commande de la partie de l'unité d'acquisition et d'affichage des images et pour le transport du signal analogique fourni par la puce de caméra CCD.

La présente invention a pour but d'éliminer les inconvénients ci-dessus de l'appareil connu en proposant un système permettant d'obtenir des images radiologiques et qui est caractérisé en ce qu'il comprend un ordinateur pour applications graphiques, une source de rayons X à paramètres d'émission contrôlables par l'ordinateur, un diaphragme de rayons X, variable et adapté pour être commandé par l'ordinateur pour limiter le faisceau de rayons X au champ nécessaire, une mire pour rayons X, déplaçable et gérée par l'ordinateur pour établir les paramètres du diaphragme et un capteur de rayons X, adapté pour recevoir les images de rayons X de l'objet à analyser, pour convertir les informations d'images en information numérique et pour envoyer ces informations numériques à l'ordinateur.

Selon une caractéristique de l'invention, le système comprend des moyens pour optimiser l'exposition d'un objet aux rayons X, qui implique la réalisation d'une double prise de vue, incluant une pré-exposition à dose réduite pour déterminer les paramètres déterminant l'exposition principale.

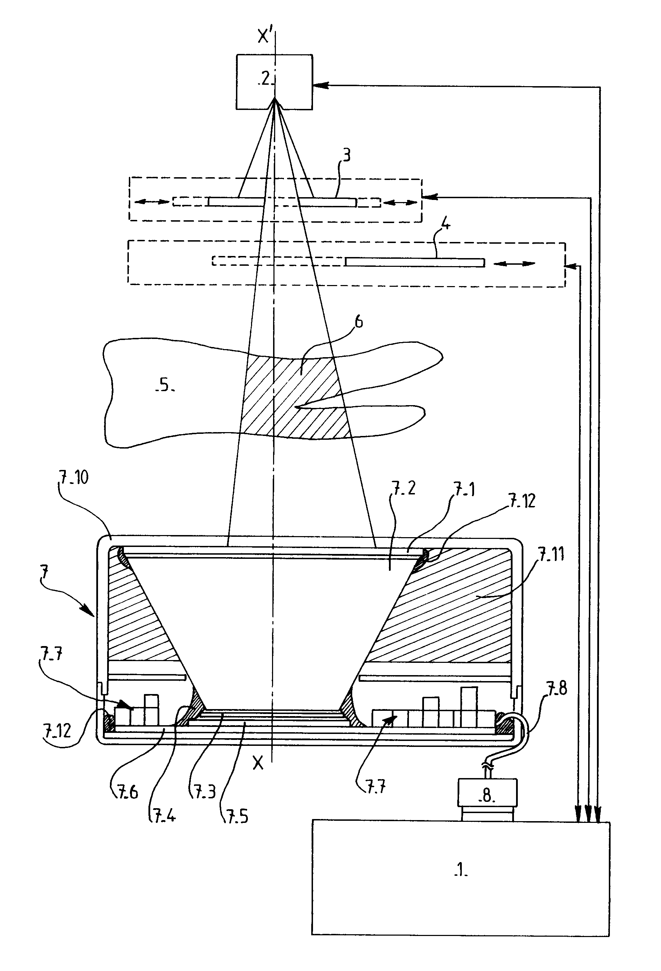

Lorsque le capteur comporte une fibre optique pour la transmission de l'image vers la surface d'une puce de caméra du type CCD, la longueur du trajet optique entre la sortie de la fibre et l'entrée dans la puce de caméra CCD présente une longueur suffisamment grande pour estomper le dessin des fibres élémentaires et suffisamment petite pour ne pas affecter la résolution de la puce de caméra CCD, dans le but d'éliminer tout effet de moirage spécifique.

La distance entre la sortie de la fibre optique et l'entrée dans la puce de caméra CCD est déterminée par un intercaleur en un matériau transparent ayant un indice de réfraction voisin de celui de la couche de protection de la puce de caméra.

Les moyens d'assemblage de la puce de caméra CCD aux autres parties optiques comportent un film liquide à faible épaisseur et un cordon périphérique de colle élastique étanche.

Selon une variante, les moyens d'assemblage de la puce de caméra CCD aux autres parties optiques comportent deux films liquides à faible épaisseur, rendus étanches à leur périphérie par un cordon de colle élastique.

Le système comprend un scintillateur disposé sur la face avant de la fibre optique, le contact optique étant réalisé par un film liquide interposé, un cordon de colle élastique étant placé sur le contour du scintillateur et constituant un moyen d'étanchéité, cette colle élastique étant de nature à ne pas entrer en réaction chimique avec les cristaux du scintillateur.

La fibre optique est fixée rigidement dans le boîtier du système par une résine dure et le support de la puce de caméra CCD est monté dans le boîtier par l'intermédiaire d'une colle élastique.

Le système comprend un agencement électronique en technologie hybride, comportant des amplificateurs de commande de la puce de caméra CCD, un générateur des séquences de commande pour les signaux spécifiques de la caméra utilisée, un circuit convertisseur analogique/numérique des signaux d'image à la sortie de la puce de caméra, un circuit de sérialisation des données ainsi obtenues, un oscillateur haute fréquence adapté pour transmettre les données par modulation de fréquence, des moyens adaptés pour séparer le courant d'alimentation des impulsions de commande et du signal d'image, ce courant et les impulsions de commande et le signal d'image étant transmis sur le même cable.

Le connecteur du capteur à l'ordinateur comporte, enfermée dans un boîtier, une mémoire EPROM ou EEPROM ou EEPROM SERIE accessible à l'ordinateur et mémorisant sous forme codifiée une ou plusieurs des informations relatives :

– à un tableau des mauvais pixels du capteur,
– à un tableau des aberrations géométriques du capteur,
– à un tableau des non-uniformités de la sensibilité du capteur,
– à un tableau de la série de fabrication et d'autres informations destinées à contrôler le marché.

L'invention sera mieux comprise, et d'autres buts, caractéristiques, détails et avantages de celle-ci apparaîtront plus clairement au cours de la description explicative qui va suivre faite en référence au dessin schématique unique illustrant un mode de réalisation du système de radiographie conforme à l'invention.

Le système de radiographie tel qu'illustré à la figure comprend :

– une unité de contrôle numérique 1 ayant une structure typique d'ordinateur à applications graphiques et permettant d'exploiter avantageusement les images reçues et également d'envoyer de signaux de commande vers d'autres sous-ensembles;
– un générateur de rayons X 2 piloté par l'unité 1 de façon que le générateur 2 fournisse une dose prescrite de rayons X;
– un élément 3 formant diaphramme rectangulaire commandé laissant passer les rayons X émis par le générateur 2 au travers de l'ouverture rectangulaire du diaphragmme, cette ouverture étant réglable en dimension par l'unité de contrôle 1;
– une mire 4 mobile sous le contrôle de l'unité 1 pouvant être interposée dans le trajet des rayons X du générateur 2 de façon que son image rayons X soit superposée avec l'image rayons X de l'objet irradié 5 qui peut être constitué par une dent; et
– un capteur 7 qui reçoit l'image rayons X de l'objet 5 et qui transmet les informations d'image à l'unité de contrôle 1.

Les moyens de commande électronique du générateur de rayons X 2 reçoivent des signaux de pilotage de l'unité de contrôle 1 de façon à contrôler la durée d'émission et/ou l'intensité d'émission et/ou la longueur d'ondes des rayons X du générateur 2.

Le générateur 2 est commandé de façon que la prise de vue puisse s'effectuer en deux temps, à savoir une phase de préexposition et une phase d'exposition principale.

Pendant la préexposition, la mire 4 est interposée dans le trajet des rayons X du générateur 2, le diaphragmme 3 est ouvert au maximum et le générateur 2 est commandé par l'unité de contrôle 1 de façon à envoyer une petite dose de rayons X (une dose minimale permettant d'obtenir une image générale sans trop de détail), ce qui permet d'obtenir deux résultats : la transparence de l'objet 5 et la position de cet objet par rapport à l'axe X-X' du générateur 2. Le premier résultat permet d'établir la dose optimale de rayons X qui sera utilisée pendant l'exposition principale. Cette tâche est confiée à une partie du logiciel spécifique qui tourne dans l'ordinateur formant l'unité de contrôle 1 et le sens d'optimisation est choisi parmi plusieurs possibilités offertes au praticien. A titre d'exemple, on peut établir comme dose optimale la dose minimale appliquée au patient qui offre sur un écran de visualisation de l'unité de contrôle 1, seize niveaux de gris bien établis sur la région d'intérêt 6 de l'objet 5. Le second résultat permet d'établir les limites latérales du faisceau de rayons X qu'il faut envoyer pendant l'exposition principale (ce qui équivaut à la protection maximale contre l'irradiation des patients). Pour établir la position de l'objet 5 par rapport à l'axe du générateur 2, la mire 4 est constituée par une plaque de support transparente aux rayons X sur laquelle est collée une seconde plaque découpée selon un dessin spécifique de dimensions déterminées, par exemple, en forme d'une croix. La matière et l'épaisseur des éléments constituant la mire 4 sont choisies afin que le contraste de l'image rayons X soit suffisant pour que le dessin puisse être connu, c'est-à-dire séparé de l'image rayons X de l'objet 5 par une partie spécifique du logiciel qui tourne dans l'unité de contrôle 1 de façon à établir la position de l'axe du générateur 2 et le degré de divergence du faisceau du générateur 2 dans le champ visuel du capteur 7.

Pendant l'exposition principale, la mire 4 est retirée du trajet des rayons X du générateur 2 et les côtés définissant l'ouverture rectangulaire du diaphragmme 3 sont positionnés par l'unité de contrôle 1 en délimitant les rayons X strictement au champ du capteur 7 ou lorsque la situation le permet au champ 6 de l'objet à analyser. Ceci a également pour avantage de réduire les rayons X parasites dûs au phénomène de réflexion ou de réémission dans l'environnement du capteur, rayons parasites qui génèrent du bruit dans l'image utile.

Le capteur d'image rayons X 7 comprend un scintillateur 7.1 qui convertit l'image rayons X en image visible qui est réduite à travers un ensemble de fibres optiques 7.2 ayant la configuration générale d'une pyramide tronquée et qui sont projetées sur la surface de la puce d'une caméra CCD 7.5. Entre la sortie de l'ensemble de fibres optiques 7.2 et la puce 7.5 de caméra CCD se trouve un intercaleur transparent 7.3 ayant une épaisseur prédéterminée. Le rôle de l'intercaleur 7.3 est d'éliminer le moirage sur l'image qui se produit par suite de la double discrimination de l'image par les éléments d'image ou pixels CCD et les fibres élémentaires optiques de l'ensemble de fibres optiques 7.2.

A la sortie d'une fibre élémentaire, la lumière est divergente de sorte que les faisceaux de lumière issus des fibres élémentaires voisines se mélangent entre eux. Par suite, compte tenu du fait que le rapport entre le diamètre d'une fibre élémentaire et la magnitude du pixel est suffisamment grand, on trouve une épaisseur suffisamment grande de l'intercaleur 7.3 afin que l'image des fibres élémentaires soient floue et suffisamment petite pour que la résolution de la puce de caméra CCD ne soit pas affectée.

L'intercaleur 7.3 est en matériau transparent ayant presque le même indice de réfraction que celui de la couche de protection de la puce de caméra 7.5. A l'interface 7.5 de la puce de caméra-intercaleur 7.3 et intercaleur 7.3-fibres 7.2 sont utilisés un ou deux fins films liquides à base d'huile anorganique ayant le même indice de réfraction que celui de l'intercaleur 7.3. Un cordon périphérique de colle élastique étanche 7.4 est formé tout autour des bords de la puce 7.5 de caméra CCD. Ce moyen de montage préserve contre les éventuels déplacements ou mouvements du capteur qui pourraient amener le remplacement de l'huile par l'air ambiant et empêche donc les pertes d'huile. L'épaisseur du film d'huile est fixe dans le temps du fait de la quantité d'huile qui est fixe. Ce moyen de montage élimine ainsi toute contrainte mécanique au niveau de la puce 7.5 de caméra CCD. Lors d'un choc mécanique, ce moyen de montage a l'avantage de se "décoller" provisoirement en formant une boule de vide qui disparaîtra aussitôt après le choc.

Le scintillateur 7.1 est disposé sur la face avant ou frontale de l'ensemble de fibres optiques 7.2 et le contact optique est réalisé par un film liquide à base d'huile anorganique interposée entre le scintillateur 7.1 et cette face avant, un cordon de colle élastique 7.12 étant placé sur le contour du scintillateur 7.1 et constituant un moyen d'étanchéité, cette colle élastique étant de nature à ne pas entrer en réaction chimique avec les cristaux du scintillateur 7.1.

Les divers éléments ci-dessus décrits du capteur 7 sont enfermés dans un boîtier en plastique 7.10 et l'élément le plus lourd, à savoir l'ensemble de fibres optiques 7.2, est fixé rigidement dans le boîtier 7.10 par une résine dure 7.11 tandis que le support 7.6 de la puce de caméra CCD 7.5 est monté dans le boîtier 7.10 par l'intermédiaire d'une colle élastique 7.12 solidarisant au moins un bord du support 7.6 à la paroi correspondante du boîtier 7.10.

Le système comprend un agencement électronique 7.7 en technologie hybride comprenant des amplificateurs de commande de la puce 7.5 de caméra CCD, un générateur des séquences de commande pour les signaux spécifiques à la caméra utilisée, un circuit convertisseur analogique/numérique des signaux d'images à la sortie de la puce de caméra 7.5, un circuit de sérialisation des données ainsi obtenues, un oscillateur haute fréquence adapté pour transmettre les données par modulation de fréquence et des moyens adaptés pour séparer le courant d'alimentation des impulsions de commande du signal d'images, ce courant et les impulsions de commande ainsi que le signal d'image étant transmis sur un même câble 7.8, tous ces éléments étant montés sur le support 7.6. Le fait que le générateur des séquences de balayage se trouve sous le support 7.6 du capteur 7 a pour avantage d'éliminer les nombreux fils normalement existants dans le cable de liaison 7.8 du capteur 7 à l'unité de contrôle 1 dans la variante ou celui-ci est placé sur l'interface spécialisée de l'ordinateur formant l'unité 1. La présence du convertisseur analogique/numérique juste à côté de la puce de caméra CCD 7.5 a pour avantage d'éliminer les problèmes de bruits spécifiques au transport de signal analogique à distance. De même, la présence du convertisseur parallèle-série (permettant d'envoyer l'information image en série) sur le support 7.6 évite d'ajouter d'autres fils assignés aux signaux numériques de sortie du convertisseur analogique/numérique. La présence sur le support 7.6 de l'oscillateur haute fréquence transmettant les données par modulation de fréquence le long du câble 7.8 élimine les problèmes qui peuvent se poser à la transmission en série des données à très grande vitesse sur un câble aussi simple que possible. Le câble 7.8 est ainsi constitué de deux fils, l'un pour la masse, l'autre d'alimentation. La séparation entre l'information d'image transmise par le capteur 7 du courant d'alimentation est effectuée par des filtres haute fréquence. Sur le câble 7.8 sont également transmis des signaux de commande provenant de l'unité de contrôle 1 sous la forme de courtes impulsions qui sont séparées par des filtres du type RC disposés dans le capteur 7 sur la plaque de support 7.6.

L'agencement électronique 7.7 est de plus protégé contre les radiations par des écrans en plomb ou de tungstène.

A l'extrémité du câble 7.8 opposé au capteur 7 est relié le connecteur du capteur 7 et dans le boîtier 8 duquel connecteur est enfermée une mémoire du type EPROM, EEPROM ou EEPROM SERIE (cette dernière étant préférée) accessible à l'ordinateur 1 et mémorisant sous forme codifiée une ou plusieurs des informations relatives :

   – à un tableau avec les coordonnées des éléments d'images ou pixels aveugles du capteur qu'il faut masquer;
   – un tableau des non-uniformités de la sensibilité du capteur afin qu'on puisse les compenser par le logiciel approprié de l'ordinateur 1;

– un tableau des aberrations géométriques du capteur qui peuvent être compensées par ce logiciel;

– un tableau de la série de fabrication et d'autres informations destinées à contrôler le marché.

L'ordinateur formant l'unité de contrôle 1 a accès à cette mémoire par l'intermédiaire du connecteur d'interface spécialisé.

**Revendications**

1. Système permettant d'obtenir des images radiologiques, caractérisé en ce qu'il comprend un ordinateur pour applications graphiques (1), une source de rayons X (2) à paramètres d'émissions contrôlables par l'ordinateur, un diaphragme de rayons X (3), variable et adapté pour être commandé par l'ordinateur pour limiter le faisceau de rayons X au champ nécessaire, une mire (4) pour rayons X, déplaçable et gérée par l'ordinateur (1) pour établir les paramètres du diaphragme (3) et un capteur (7) de rayons X, adapté pour recevoir les images de rayons X de l'objet à analyser (5), pour convertir les informations d'images en informations numériques et pour envoyer ces informations numériques à l'ordinateur (1).

2. Système selon la revendication 1, caractérisé en ce qu'il comprend des moyens pour optimiser l'exposition d'un objet (5) aux rayons X, qui implique la réalisation d'une double prise de vue, incluant une pré-exposition à dose réduite pour déterminer les paramètres déterminant l'exposition principale.

3. Système selon la revendication 1, caractérisé en ce que, lorsque le capteur comporte un ensemble de fibres optiques (7.2) pour la transmission de l'image vers la surface d'une puce de caméra du type CCD (7.5), la longueur du trajet optique entre la sortie de la fibre et l'entrée dans la caméra CCD présente une longueur suffisamment grande pour estomper le dessin des fibres élémentaires et suffisamment petite pour ne pas affecter la résolution de la puce de caméra CCD, dans le but d'éliminer tout effet de moirage spécifique.

4. Système selon la revendication 3, caractérisé en ce que la distance entre la sortie de l'ensemble de fibres optiques (7.2) et l'entrée dans la puce de caméra CCD (7.5) est déterminée par un intercaleur (7.3) en un matériau transparent ayant un indice de réfraction voisin de celui de la couche de protection de la puce de caméra.

5. Système selon la revendication 3, caractérisé en ce que les moyens d'assemblage de la puce de caméra CCD aux autres parties optiques comportent un film liquide à faible épaisseur et un cordon périphérique de colle élastique étanche.

6. Système selon l'une des revendications 3 ou 4, caractérisé en ce que les moyens d'assemblage de la puce de caméra CCD aux autres parties optiques comportent deux films liquides à faible épaisseur, rendus étanches à leur périphérie par un cordon de colle élastique (7.4).

7. Système selon l'une des revendications précédentes, caractérisé en ce qu'il comprend un scintillateur (7.1) disposé sur la face avant de l'ensemble de fibres optiques (7.2), le contact optique étant réalisé par un film liquide interposé, un cordon de colle élastique (7.12) étant placé sur le contour du scintillateur et constitue un moyen d'étanchéité, cette colle élastique étant de nature à ne pas entrer en réaction chimique avec les cristaux du scintillateur.

8. Système selon l'une des revendications 3 à 7, caractérisé en ce que la fibre optique (7.2) est fixée rigidement dans le boîtier (7.10) du système par une résine dure (7.11) et en ce que le support (7.6) de la puce de caméra CCD (7.5) est monté dans le boîtier (7.10) par l'intermédiaire d'une colle élastique (7.12).

9. Système selon l'une des revendications précédentes, caractérisé en ce qu'il comprend un agencement électronique (7.7) en technologie hybride, comportant des amplificateurs de commande de la puce de caméra CCD, un générateur des séquences de commande pour les signaux spécifiques de la caméra utilisée, un circuit convertisseur analogique/numérique des signaux d'image à la sortie de la puce de caméra, un circuit de sérialisation des données ainsi obtenues, un oscillateur haute fréquence adapté pour transmettre les données par modulation de fréquence, des moyens adaptés pour séparer le courant d'alimentation des impulsions de commande et du signal d'image, ce courant et les impulsions de commande et le signal d'image étant transmis sur le même câble (7.8).

10. Système selon l'une des revendications précédentes, caractérisé en ce que le connecteur du capteur (7) à l'ordinateur (1) comporte, enfermée dans un boîtier (8), une mémoire EPROM ou EEPROM ou EEPROM SERIE accessible à l'ordinateur et mémorisant sous forme codifiée une ou plusieurs des informations relatives :

– à un tableau des mauvais pixels du capteur,

– à un tableau des aberrations géométriques du capteur,

– à un tableau des non-uniformités de la sensibilité du capteur,

– à un tableau de la série de fabrication et d'autres informations destinées à contrôler le marché.

## RAPPORT DE RECHERCHE EUROPEENNE

Office européen
des brevets

Numero de la demande

EP   92 40 0819

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| P,A | FR-A-2 657 694 (GENERAL ELECTRIC COMPANY)<br><br>* page 2, ligne 7 - ligne 30 *<br>* page 3, ligne 17 - ligne 24 *<br>* page 5, ligne 6 - page 7, ligne 3 *<br>* page 8, ligne 21 - ligne 25; figures * | 1,3,5,8,9 | A61B6/14<br>G02B6/42 |
| D,A | FR-A-2 578 737 (F. MOUYEN)<br>* page 1, ligne 20 - page 2, ligne 2 *<br>* page 2, ligne 29 - page 3, ligne 27; figure * | 1,8 | |
| A | DE-A-3 641 992 (PHILIPS)<br>* colonne 2, ligne 49 - ligne 64 *<br>* colonne 5, ligne 6 - ligne 41 * | 2 | |
| A | REVIEW OF SCIENTIFIC INSTR.<br>vol. 50, no. 11, Novembre 1979,<br>pages 1416 - 1420;<br>R. C. GAMBLE ET AL.: 'Linear position-sensitive x-ray detector incorporating a self-scanning photodiode array.'<br>* colonne 2, ligne 23 - ligne 31 *<br>* colonne 4, ligne 47 - colonne 5, ligne 14 *<br>* colonne 6, ligne 29 - colonne 7, ligne 11 *<br>* colonne 9, ligne 11 - ligne 25; figure 1 * | 9,10 | |
| A | EP-A-0 413 043 (SIEMENS AG)<br>* colonne 3, ligne 47 - colonne 4, ligne 22 *<br>* colonne 5, ligne 7 - ligne 43; figures 3,4 * | 1 | |

**DOMAINES TECHNIQUES RECHERCHES (Int. Cl.5)**

A61B
H01L
G02B
G01T

Le présent rapport a été établi pour toutes les revendications

| Lieu de la recherche | Date d'achèvement de la recherche | Examinateur |
|---|---|---|
| LA HAYE | 03 JUILLET 1992 | FONTENAY P.H. |

EPO FORM 1503 03.82 (P0402)